(19) **Europäisches Patentamt / European Patent Office / Office européen des brevets**

(11) **EP 1 424 080 B1**

(12) **EUROPEAN PATENT SPECIFICATION**

(45) Date of publication and mention
of the grant of the patent:
**16.02.2011 Bulletin 2011/07**

(21) Application number: **02758814.4**

(22) Date of filing: **08.08.2002**

(51) Int Cl.:
*A61K 45/06* (2006.01)     *A61K 9/52* (2006.01)
*A61K 31/4535* (2006.01)     *A61K 31/58* (2006.01)
*A61K 38/00* (2006.01)     *A61P 3/14* (2006.01)
*A61P 9/00* (2006.01)     *A61P 13/08* (2006.01)
*A61P 15/00* (2006.01)     *A61P 15/08* (2006.01)
*A61P 15/12* (2006.01)     *A61P 19/00* (2006.01)
*A61P 25/22* (2006.01)     *A61P 25/28* (2006.01)
*A61P 35/00* (2006.01)     *A61P 43/00* (2006.01)

(86) International application number:
**PCT/JP2002/008130**

(87) International publication number:
**WO 2003/015820 (27.02.2003 Gazette 2003/09)**

(54) **GNRH AGONIST COMBINATION DRUGS**

GNRH-AGONISTISCHE KOMBINATIONSMITTEL

MEDICAMENTS COMBINES D'AGONISTE DE LA GNRH

(84) Designated Contracting States:
**AT BE BG CH CY CZ DE DK EE ES FI FR GB GR IE IT LI LU MC NL PT SE SK TR**

(30) Priority: **10.08.2001 JP 2001244616**

(43) Date of publication of application:
**02.06.2004 Bulletin 2004/23**

(73) Proprietor: **Takeda Pharmaceutical Company Limited
Osaka (JP)**

(72) Inventors:
• **FURUYA, Shuichi
Higashinada-ku, Kobe-shi, Hyogo (JP)**
• **KUSAKA, Masami
Kobe-shi, Hyogo 651-2102 (JP)**

(74) Representative: **Keller, Günter et al
Lederer & Keller
Patentanwälte
Prinzregentenstrasse 16
80538 München (DE)**

(56) References cited:
**EP-A- 1 382 350     WO-A1-00/03979
WO-A1-01/30764     WO-A1-01/77107
WO-A1-86/01105     WO-A1-97/27863
WO-A1-97/40846     WO-A1-99/54309**

**WO-A2-00/07576     WO-A2-99/08668
JP-A- 2002 080 458     JP-A- 2002 234 843
JP-A- 2002 241 268     JP-A- 2002 241 377
US-A- 4 472 382**

• **MICHAUD L.B. ET AL.: 'Endocrine therapy of metastatic breast cancer' SEMINARS IN BREAST DISEASE vol. 3, no. 2, 2000, pages 100 - 111, XP002960247**
• **MICHAUD L.B. ET AL.: 'Complete estrogen blockade for the treatment of metastatic and early breast cancer' DRUGS & AGING vol. 16, no. 4, 2000, pages 261 - 271, XP002960248**
• **TRAYNOR A.: 'Recent advances in hormonal therapy for cancer' CURRENT OPINION IN ONCOLOGY vol. 7, no. 6, 1995, pages 572 - 581, XP000574188**
• **DOWLING A.J. ET AL.: 'Systematic treatment for prostate cancer' CANCER TREATMENT REVIEWS vol. 24, no. 4, 1998, pages 283 - 301, XP000910517**
• **PICKERSGILL A.: 'GnRH agonists and add-back therapy: is there a perfect combination?' BR. J. OBSTET. GYNECOL. vol. 105, no. 5, 1998, pages 475 - 485, XP002960249**

**(Cont. next page)**

- SURREY E.S. ET AL.: 'Effects of sodium etidronate in combination with low-dose norethindrone in patients administered a long-acting GnRH agonist: A preliminary report' OBSTETETRICS AND GYNECOLOGY vol. 81, no. 4, 1993, pages 581 - 586, XP002960250
- MUKHERJEE T. ET AL.: 'A randomized, placebo-controlled study on the effect of cyclic intermittent etidronate therapy on the bone mineral density changes associated with six months of gonadotropin-releasing hormone agonist treatment' AM. J. OBSTET. GYNECOL. vol. 175, no. 1, 1993, pages 105 - 109, XP002960251
- GAMBACCIANI M. ET AL.: 'Ipriflavone prevents the bone mass reduction in premenopausal women treated with gonadotropin hormone-releasing hormone agonists' BONE AND MINERAL vol. 26, no. 1, 1994, pages 19 - 26, XP002960252
- GAMBACCIANI M. ET AL.: 'Ipriflavone prevents the loss of bone mass in pharmacological menopause induced by GnRG-agonist' CALCIF. TISSUE INT. vol. 61, no. SUPPL. 1, 1997, pages S15 - S18, XP002960253
- DATABASE CAPLUS [Online] (COLUMBUS, OHIO, USA) DN 129:35986 BLACKLEDGE G.R.P. ET AL.: 'Emerging drugs in prostate cancer', XP002960254 Database accession no. 1998: 292127 & EMERGING DRUGS vol. 3, 1998, pages 303 - 315
- DATABASE CAPLUS [Online] (COLUMBUS, OHIO, USA) SAKAMOTO S. ET AL.: 'Prevention of osteopenia induced with a gonadotropin-releasing hormone agonist in rats', XP002960255 Database accession no. 252762 & CALCIF. TISSUE. INT. vol. 65, no. 2, 1999, pages 152 - 155
- YAHALOM D. ET AL.: 'Hexapeptide and cylic pentapeptide endothelin antagonists directly activate pituitary gonadotropin-releasing hormone receptors' MOL. PHARM. vol. 57, no. 4, 2000, pages 718 - 724, XP002960256
- CLARK J.W. ET AL: 'EFFECTS OF TYROSINE KINASE INHIBITORS ON THE PROLIFERATION OF HUMAN BREAST CANCER CELL LINES AND PROTEINS IMPORTANT IN THE RAS SIGNALING PATHWAY' INTERNATIONAL JOURNAL OF CANCER, JOHN WILEY & SONS, INC, UNITED STATES, SWITZERLAND, GERMANY vol. 65, no. 2, 17 January 1996, pages 186 - 191, XP001011272 ISSN: 0020-7136
- EL-ZARRUK A.A. ET AL: 'The anti-proliferative effects of tyrosine kinase inhibitors towards tamoxifen-sensitive and tamoxifen-resistant human breast cancer cell lines in relation to the expression of epidermal growth factor receptors (EGF-R) and the inhibition of EGF-R tyrosine kinase' CANCER LETTERS, NEW YORK, NY, US vol. 142, no. 2, 03 August 1999, pages 185 - 193, XP002192996 ISSN: 0304-3835
- PROSTATE CANCER TRIALISTS COLLABORATIVE GROUP: "Maximum androgen blockade in advanced prostate cancer: an overview of the randomised trials" LANCET THE, LANCET LIMITED. LONDON, GB, vol. 355, no. 9214, 29 April 2000 (2000-04-29), pages 1491-1498, XP005018479 ISSN: 0140-6736
- GEORGE DANIEL J. ET AL: 'SUSTAINED IN VIVO REGRESSION OF DUNNING H RAT PROSTATE CANCERS TREATED WITH COMBINATIONS OF ANDROGEN ABLATION AND TRK TYROSINE KINASE INHIBITORS, CEP-751 (KT-6587) OR CEP-701 (KT-5555)' CANCER RESEARCH, AMERICAN ASSOCIATION FOR CANCER RESEARCH, BALTIMORE, MD., US vol. 59, 15 May 1999, pages 2395 - 2401, XP008070906 ISSN: 0008-5472
- RAO G.S. ET AL: 'Radiosensitization of human breast cancer cells by a novel ErbB family receptor tyrosine kinase inhibitor' INTERNATIONAL JOURNAL OF RADIATION: ONCOLOGY BIOLOGY PHYSICS, PERGAMON PRESS, USA vol. 48, no. 5, 01 December 2000, pages 1519 - 1528, XP002257052 ISSN: 0360-3016
- GEE J. ET AL: 'The EGFR-selective tyrosine kinase inhibitor ZD1839 ('Iressa') is an effective inhibitor of tamoxifen-resistant breast cancer growth' EUROPEAN JOURNAL OF CANCER, PERGAMON PRESS, OXFORD, GB vol. 37, 01 April 2001, page S261, XP004478210 ISSN: 0959-8049
- JACKSON I.M. ET AL: 'LHRH analogues in the treatment of cancer' CANCER TREATMENT REVIEWS, SAUNDERS, US vol. 16, no. 3, 01 September 1989, pages 161 - 175, XP023271011 ISSN: 0305-7372

Remarks:
The file contains technical information submitted after the application was filed and not included in this specification

**EP 1 424 080 B1**

**Description**

FIELD OF THE INVENTION

The present invention relates to a pharmaceutical composition which is a combination of a GnRH agonist and a certain drug.

BACKGROUND OF THE INVENTION

A luteinizing hormone-releasing hormone which is known as an LHRH (or GnRH) is secreted from a hypothalamus and bound to a pituitary receptor. As a result, an LH (luteinizing hormone) and an FSH (follicle-stimulating hormone) are secreted and act on a gonad whereby synthesizing steroidal sex hormones. A continuous administration of a compound having a potent luteinizing hormone-releasing hormone activity results in a reduction in the number of available receptors, which reduces the formation of gonad-derived steroidal sex hormones. Utilizing such a behavior, a compound having a GnRH activity is applied clinically as a drug for treating sex hormone-dependent diseases such as prostate cancer, benign prostatic hypertrophy, endometriosis, hysteromyoma, uterine fibrosis, precocious puberty, breast cancer and the like.

US Patent US 4.472.382 discloses a method of treating prostate adenocarcinoma, prostate benign hypertrophia and some other related diseases with luteinising hormone releasing hormone (GnRH) analogues.

WO 86/01105 discloses a method for treating sex steroid dependent cancers in a warm-blooded animal which comprises administering a pharmaceutically effective amount of an antiandrogen and/or an antiestrogen and/or at least one inhibitor of sex steroid biosynthesis to an animal whose hormone output of the testes or ovaries is blocked.

I. M. Jackson et al. (Cancer Treatment Reviews 16, 161-175, 1989) provides an overview on early evidences of the efficacy and safety of treatment with luteinising hormone releasing hormone (GnRH) analogues for the treatment of hormone-dependent cancers. The document mentions, among others, agents buserelin and leuprolide.

J. W. Clark et al. (Int. J. Cancer 65, 186-191, 1996) demonstrate, that tyrosine kinase inhibitors (herbimycin A and genistein) can inhibit proliferation of breast cancer cell lines SKBR3 and MCF-7, accompanied by inhibition of signal transduction steps potentially mediated through ras.

A. A. E1-Zarruk et al. (Cancer Letters 142, 185-193, 1999) report an anti-proliferative effect of the tyrosine kinase inhibitors against human breast cancer cell line ZR-PR-LT and its variants ZR-75-9a1 and ZR-PR-LT. The determined $IC_{50}$ values range from 0.13 to 2.9 $\mu$M depending on the inhibitor or cell line.

G. S. Rao et al. (Int. J. Radiation Oncology Biol. Phys. 48, 1519-1528, 2000) investigated the effect of agent CI-1033, an ErbB tyrosine kinase inhibitor, in combination with ionizing radiation on human breast cancer cells. The results revealed that CI-1033 shows a potent growth inhibition alone and synergistic effects when combined with ionizing radiation.

J. Gee et al. (European Journal of Cancer, 37, S261) reveal that the EGFR-selective tyrosine kinase inhibitor ZD1839 ("Iressa") is an effective inhibitor of tamoxifen-resistant breast cancer growth.

Possibilities of endocrine therapy of metastatic breast cancer are further described in the review article by L. Boehnke Michaud et al. (Seminars in breast disease, 3, 100-111, 2000). The article describes, among others, use of GnRH agonists goserelin, leuprolide and buserelin for treatment of human breast cancer.

Leuprolide acetate and goserelin acetate are further mentioned in the review article by A. J. Dowling et al. (Cancer Treatment Reviews 24, 283-301, 1998) as agents for treatment of adenocarcinoma of the prostate.

An overview of the randomized clinical trials published by O. Dalesio et al. (The Lancet 355, 1491-1498, 2000) reveals that addition of an antiandrogen treatment to androgen suppression improved the 5-year survival of patients with an advanced prostate cancer by about 2 or 3%, whereby the true size of the benefit runs from about 0% to about 5%.

EP 1382350 discloses peptidic compounds acting as GnRH agonists or antagonists which can prevent a postoperative recurrence of premenopausal breast cancer without severe side effects. The agents can be applied in form of sustained-release preparations and can also be administered in combination with a concomitant drug.

WO 01/77107 discloses a heterocyclic compound acting as a growth factor receptor tyrosine kinase (particularly HER2) inhibitor. This compound can be therefore employed for prevention or treatment of tyrosine kinase-dependent diseases in mammals. The compound can be also administered in combination with other anti-cancer agents.

WO 99/08668 discloses a method of treating prostate cancer by coadministering a tyrosine kinase inhibitor such as an indolocarbazole and a chemical castration agent. The chemical castration agents mentioned therein also include GnRH antagonists such as leuprolide acetate.

D. J. George et al. (Cancer Research 59, 2395-2401, 1999) show that the treatment with indolocarbazole analogue CEP-701, when combined with administration of leuprolide to induce androgen ablation produces a significantly greater tumor regression than either therapy alone, with no signs of regrowth. As a test system for this study Dunning R-3327 H rat prostate cancer was chosen.

3

DISCLOSURE OF THE INVENTION

An objective of the present invention is to provide a pharmaceutical composition and a preventative or therapeutic method capable of improving preventative or therapeutic effect of a GnRH agonist on various diseases such as prostate cancer as well as improving QOL (quality of life).

The present inventors have studied intensively to achieve the above objective and, as a result, have found that, by using a GnRH agonist in combination with a SERM drug or a receptor-type tyrosine kinase inhibitor it is possible to improve a preventative or therapeutic effect of a GnRH agonist on various diseases prostate cancer markedly, and also to improve QOL markedly. The present inventors have further studied based on this finding, and completed the present invention.

That is, the present invention relates to:

[1] A pharmaceutical composition for use in preventing or treating prostate cancer, postoperative recurrence of prostate cancer or metastasis of prostate cancer which is a combination of a GnRH agonist and a receptor-type tyrosine kinase inhibitor, wherein the GnRH agonist is an acetate of the peptide represented by the formula:

5-oxo-Pro-His-Trp-Ser-Tyr-DLeu-Leu-Arg-Pro-NH-$C_2H_5$,

and the receptor-type tyrosine kinase inhibitor is GW-2016;

[2] The combination of a GnRH agonist and a receptor-type tyrosine kinase inhibitor for use in preventing or treating prostate cancer, postoperative recurrence of prostate cancer, or metastasis of prostate cancer, wherein the GnRH agonist is an acetate of the peptide represented by the formula:

5-oxo -Pro-His-Trp-Ser-Tyr-DLeu-Leu-Arg-Pro-NH-$C_2H_5$,

and the receptor-type tyrosine kinase inhibitor is GW-2016;

[3] Use of a combination of a GnRH agonist and a receptor-type tyrosine kinase inhibitor for the manufacture of a medicament for the prevention or treatment of prostate cancer, postoperative recurrence of prostate cancer, or metastasis of prostate cancer, wherein the GnRH agonist is an acetate of the peptide represented by the formula:

5-oxo-Pro-His-Trp-Ser-Tyr-DLeu-Leu-Arg-Pro-NH-$C_2H_5$,

and the receptor-type tyrosine kinase inhibitor is GW-2016;

[4] The pharmaceutical composition according to the above [1] wherein the GnRH agonist is used as a sustained release preparation or an implant;

[5] The pharmaceutical composition according to the above [4], wherein the sustained release preparation is a sustained release microcapsule; etc.

Examples of the GnRH agonist include a GnRH agonist effective in hormone-dependent diseases, in particular, sex hormone-dependent cancer (e.g., prostate cancer, uterine cancer, breast cancer, pituitary tumor, etc.), sex hormone-dependent diseases such as prostatic hypertrophy, endometriosis, hysteromyoma, precocious puberty, dysmenorrhea, amenorrhea, premenstrual syndrome, multilocular ovary syndrome, postoperative recurrence of the above-mentioned cancer, dwarfism, Alzheimer's disease, menopausal syndrome, equivocal complaint, metastasis of the above-mentioned cancer, calcium/phosphorus bone dysbolism, etc., as well as contraception (or infertility when utilizing a rebound effect after discontinuing the drug). Further, those also exemplified are GnRH agonists effective against a non-sex hormone-dependent but GnRH sensitive benign or malignant tumor, etc.

Specific examples of the GnRH agonist include an acetate of a peptide represented by 5-oxo-Pro-His-Trp-Ser-Tyr-DLeu-Leu-Arg-Pro-NH-$C_2H_5$ (leuprorelin acetate TAKEDA CHEMICAL INDUSTRIES, LTD.).

The above GnRH agonist, preferably leuprorelin acetate can be administered orally in the form of optionally sugar-coated tablets, capsules, elixirs, sustained release preparations, and the like, or parenterally in the form of injectable preparations such as aseptic solutions, suspensions, sustained release preparations (especially sustained release microcapsules) in water or other pharmaceutically acceptable liquids, implants (molded by using a biodegradable polymer as a base, encapsulated in a cylinder of a biocompatible metal such as titanium which releases an active ingredient at a constant rate), injectable preparations formed by dissolving or dispersing a biodegradable polymer and a drug in an organic solvent capable of being administered to a living body, as well as nasal preparations such as solutions or suspensions, etc. with a sustained release preparation being preferred and a sustained release injectable preparation being especially preferred. When the sustained release preparation is a sustained release microcapsule, it is preferably a long term sustained release microcapsule which releases a GnRH agonist or antagonist over a period of 2 months or longer.

Leuprorelin acetate, can be admixed together with a physiologically acceptable known carrier, flavor, excipient, vehicle, preservative, stabilizer, binder, etc. in a unit dosage form generally required in practicing a pharmaceutical preparation to obtain the above preparation.

As additives which may be incorporated into tablets or capsules, there are, for example, binders such as gelatin, corn starch, tragacanth and gum arabic, excipients such as crystalline cellulose, swelling agents such as corn starch, gelatin, alginic acid, etc., lubricants such as magnesium stearate, sweeteners such as sucrose, lactose or saccharin, flavors such as peppermint, Akamono oil, cherry, etc. When a unit dosage form is a capsule, a liquid carrier such as a fat may further be incorporated in addition to the above-mentioned materials. An aseptic composition for injection can be prepared in accordance with a conventional pharmaceutical practice such as dissolution or suspension of an active component, a naturally-occurring vegetable oil such as sesame oil and palm oil in a vehicle such as injectable water. Examples of an injectable aqueous solution include physiological saline, an isotonic solution containing glucose or other auxiliary agents (for example, D-sorbitol, D-mannitol, sodium chloride, etc.) and the like, which may be used in combination with a suitable solubilizer such as alcohol (for example, ethanol), polyalcohol (for example, propylene glycol, polyethylene glycol), nonionic surfactant (for example, polysorbate 80(TM), HCO-50), etc. As an oily liquid, there is, for example, sesame oil, soybean oil, etc. which may be used in combination with a solubilizer such as benzyl benzoate, benzyl alcohol and the like.

In addition, a buffer (for example, phosphate buffer, sodium acetate buffer), a soothing agent (for example, benzalkonium chloride, procaine hydrochloride, etc.), a stabilizer (for example, human serum albumin, polyethylene glycol, etc.), a preservative (for example, benzyl alcohol, phenol, etc.), an antioxidant may also be incorporated. The injectable preparation thus prepared is then filled usually in a suitable tightly-sealable container such as an ampoule or a vial.

The above-mentioned sustained release preparation (especially a sustained release microcapsule) containing the GnRH agonist (preferably leuprorelin acetate) can be produced by a method known per se, such as those described in JP 60-100516 A, JP 62-201816 A, JP 4-321622 A, JP 6-192068 A, JP 9-132524 A, JP 9-221417 A, JP 11-279054 A, WO 99/360099 and the like.

Among the above sustained release preparations, preferably, "a long term sustained release microcapsule capable of performing a zero-dimensional release of a physiologically active substance over a period of 2 months or longer" described in JP 4-321622 A is used.

Hereinafter, one example of processes for producing the above sustained release microcapsule will be illustrated.

First, a GnRH agonist (preferably leuprorelin acetate) is dissolved in water in an amount of about 20% to 70% (W/W), preferably, 25 - 65%(W/W), more preferably 35 - 60%(W/W) and, if necessary, a drug-retaining agent such as gelatin or a basic amino acid is dissolved or suspended therein to form an inner aqueous phase solution.

To this inner aqueous phase solution may be added, as a pH adjustor for maintaining stability and solubility of a GnRH agonist (preferably leuprorelin acetate), carbonic acid, acetic acid, oxalic acid, citric acid, phosphoric acid, hydrochloric acid, sodium hydroxide, arginine, lysine or a salt thereof, or the like. To this solution may also be added, as a stabilizer for a GnRH agonist (preferably leuprorelin acetate), albumin, gelatin, citric acid, sodium ethylenediamine tetraacetic acid, dextrin, sodium hydrogen sulfite, a polyol compound such as polyethylene glycol, or, as a preservative, a conventionally employed ethyl p-oxybenzoate (methyl paraben, propyl paraben, and the like), benzyl alcohol, chlorobutanol, thimerosal, and the like.

The inner aqueous phase solution thus obtained is added to a polymer-containing solution (oil phase), and then emulsified to obtain a W/O-type emulsion. A known dispersion method can be employed for the emulsification and, as the emulsification operation, there are, for example, an intermittent shaking method, a method with a mixer such as a propeller stirrer, turbine stirrer, etc., colloid mill method, homogenizer method, ultrasonic irradiation method, and the like.

Then, the W/O-type emulsion thus prepared is subjected to a microencapsulating step. As such a step, an in-water drying method or a phase separation method can be employed. When an in-water drying method is employed to prepare microcapsules, the W/O emulsion is further added to a third aqueous phase to form a three phase emulsion of W/O/W type, and thereafter the solvent in the oil phase is evaporated to prepare microcapsules.

An emulsifier may be added to the aqueous phase of the above outer phase. The emulsifier may be any one which is generally capable of forming a stable O/W emulsion and examples thereof include anionic surfactants (sodium oleate, sodium stearate, sodium lauryl sulfate, and the like), nonionic surfactants (polyoxyethylene sorbitan fatty acid esters [Tween 80, Tween 60, Atras Powder], polyoxyethylene castor oil derivatives [HCO-6, HCO-50, Nikko Chemicals], and the like), or polyvinylpyrrolidone, polyvinyl alcohol, carboxymethylcellulose, lecithin, gelatin, and the like. They can be used alone or in combination with each other. When using them, their concentration can be appropriately selected from the range of about 0.01% to 20% and, more preferably, they can be used within the range of about 0.05% to 10%.

For evaporation of the solvent in the oil phase, a conventional method can be employed. As such a method, the evaporation can be performed by stirring with a propeller stirrer or a magnetic stirrer while gradually reducing pressure, or by a rotary evaporator with adjusting vacuum conditions. In such a case, the time period required can be reduced by warming the W/O/W emulsion gradually once the solidification of the polymer has been advanced to a certain extent for the purpose of ensuring removal of the solvent.

The microcapsules thus obtained are recovered, for example, by centrifugation or filtration and then washed with a distilled water several times repeatedly to remove materials adhered on the surfaces of microcapsules such as the free GnRH agonist (preferably leuprorelin acetate), drug-retaining agent, emulsifier and the like, and then dispersed again, for example, in a distilled water, and then lyophilized. At this time, an aggregation-preventing agent (for example, mannitol, sorbitol, lactose, glucose, and the like) may be added. If necessary, the removal of water and organic solvents in the microcapsules can further be performed under reduced pressure to ensure complete removal.

When microcapsules are prepared by a phase separation method, a coacervation agent is added slowly to the W/O emulsion with stirring to precipitate and solidify the polymer.

As the coacervation agent, any compounds selected from polymers, minerals or vegetable oils which are capable of being miscible with a solvent for the polymer and do not dissolve the polymer for encapsulation, and examples thereof include silicone oil, sesame oil, soybean oil, corn oil, cottonseed oil, coconut oil, linseed oil, mineral oil, n-hexane, n-heptane, and the like. They can also be used as a mixture of two or more thereof.

The microcapsules thus obtained are recovered by filtration, washed repeatedly for example with heptane, etc. to remove the coacervation agent. Further, they are subjected to removal of free drugs and solvents by the same method as that in the in-water drying method. An aggregation-preventing agent may be added to prevent aggregation between particles with each other during the washing.

The above-obtained microcapsules may be ground gently if necessary, and then sieved to remove any too large microcapsules. Desirably, the particle size of microcapsules is within the range of about 0.5 to 1000 $\mu$m, more preferably about 2 to 500 $\mu$m in terms of the average particle size. In case of using as an injectable suspension, the particle size of microcapsules may be within such a range that they satisfy the desired dispersibility and passability through a needle, for example, about 2 to 100 $\mu$m.

Examples of the above polymer include biodegradable polymers, such as a polymer or a copolymer having a free carboxyl group which is synthesized from one or more $\alpha$-hydroxycarboxylic acids including $\alpha$-hydroxymonocarboxylic acids (e.g., glycolic acid, lactic acid, etc.), $\alpha$-hydroxydicarboxylic acids (e.g., malic acid), $\alpha$-hydroxytricarboxylic acids (e.g., citric acid) and the like, or a mixture thereof; a poly($\alpha$-cyanoacrylate); a polyamino acid (e.g., poly($\gamma$-benzyl-L-glutamic acid), etc.); a maleic anhydride copolymer (e.g., styrene-maleic acid copolymer, etc.); and the like.

The polymerization mode of monomers may be any of random, block and graft polymerization. When the above $\alpha$-hydroxymonocarboxylic acids, $\alpha$-hydroxydicarboxylic acids or $\alpha$-hydroxytricarboxylic acids have optically active centers in their molecules, they may be any of D-, L-, DL-forms. Among them, the preferred one is a lactic acid-glycolic acid polymer (hereinafter sometimes referred to as poly(lactide-co-glycolide), poly(lactic acid-co-glycolic acid) or lactic acid-glycolic acid copolymer, and, unless otherwise stated, generally inclusive a homopolymer (polymer) or copolymer of lactic acid and glycolic acid; lactic acid homopolymer is also referred to as lactic acid polymer, polylactic acid, polylactide, etc., while glycolic acid homopolymer is also referred to as glycolic acid polymer, polyglycolic acid, polyglycolide, etc.), and the like. A lactic acid-glycolic acid polymer is further preferred, with a lactic acid-glycolic acid polymer having a free carboxylate group on its terminal being more preferred.

The biodegradable polymer may be a salt. Examples of the salt include a salt with an inorganic base (e.g., alkaline metal such as sodium and potassium, alkaline earth metal such as calcium and magnesium) or with an organic base (e.g., organic amines such as triethylamine, basic amino acid such as arginine), or with a transition metal (e.g., zinc, iron, copper) as well as a complex salt.

When a lactic acid-glycolic acid polymer is used as the biodegradable polymer, the composition ratio (% by mole) is preferably about 100/0 to about 40/60, more preferably about 100/0 to about 50/50. In the case of a long term sustained release microcapsule capable of performing a zero-dimensional release of a physiologically active substance over a period of 2 months or longer, the lactic acid homopolymer whose composition ratio is 100/0 is preferably used.

The optical isomer ratio of the lactic acid which is one of the minimum repeating units of the "lactic acid-glycolic acid polymer", when represented as a D-form/L-form (mol/mol %), is preferably about 75/25 to about 25/75. Those having such a D-form/L-form (mol/mol %) within the range of about 60/40 to about 30/70 are especially employed frequently.

The weight average molecular weight of the "lactic acid-glycolic acid polymer" is usually about 3,000 to about 100,000, preferably about 3,000 to about 60,000, more preferably about 3,000 to about 50,000.

The dispersion degree (weight average molecular weight/number average molecular weight) is usually about 1.2 to about 4.0, more preferably about 1.5 to 3.5.

The amount of the free carboxyl groups in the "lactic acid-glycolic acid polymer" per unit mass (gram) of the polymer is usually preferably about 20 to about 1000 $\mu$mol (micromole), especially preferably about 40 to about 1000 $\mu$mol (micromole).

The weight average molecular weight, the number average molecular weight and the dispersion degree mentioned above are the molecular weights as those of polystyrene and the correspondingly calculated dispersion degree on the basis of the measurement by a gel permeation chromatography (GPC) using as standards the 15 monodisperse polystyrenes having weight average molecular weights of 1,110,000, 707,000, 455,645, 354,000, 189,000, 156,055, 98,900, 66,437, 37,200, 17,100, 9,830, 5,870, 2,500 and 1,303, 504. The measurement is performed using a high performance GPC device (TOSOH CORP., HLC-8120GPC, detection by differential refractory index) together with a GPC column KF804L x 2 (SHOWA DENKO K.K.) and chloroform as a mobile phase. The flow rate is 1 ml/min.

The above amount of free carboxyl group is one measured by a labeling method (herein after referred to as a labeling method-based carboxyl group level). Specifically, when exemplified in the case of a polylactic acid, W mg of the polylactic acid is dissolved in 2 ml of a mixture of 5N hydrochloric acid/acetonitrile (v/v=4/96). To the mixture were added 2 ml of a 0.01M o-nitrophenylhydrazine hydrochloride (ONPH) solution (5N hydrochloric acid/acetonitrile/ethanol = 1.02/35/15) and 2 ml of a 0.15M 1-ethyl-3-(3-dimethylaminopropyl)carbodiimide hydrochloride solution (pyridine/ethanol = 4v/96v), followed by reaction at 40°C for 30 minutes. Then, the solvent is distilled off, and the residue is washed with water (4 times) and dissolved in 2 ml of acetonitrile. To the solution was added 1 ml of a 0.5 mol/l ethanolic solution of potassium hydroxide, and the mixture was reacted at 60°C for 30 minutes. The reaction mixture is diluted with a 1.5N aqueous solution of sodium hydroxide to make up to Y ml, which is subjected to the measurement of the absorbance A at 544 nm (/cm) using a 1.5N aqueous solution of sodium hydroxide as a reference control. On the other hand, when an aqueous solution of DL-lactic acid is used as a standard, whose free carboxyl group level C mol/L is determined by alkali titration, and the absorbance at 544 nm of a DL-hydrazide lactate obtained by an ONPH labeling method is designated as B (/cm); the molar amount of the free carboxyl groups per unit mass (gram) of the polymer can be calculated as follows:

$$[COOH](mol/g) = (AYC)/(WB)$$

The "carboxyl group level" can also be determined by dissolving a biodegradable polymer in a toluene-acetone-methanol mixed solvent and titrating the carboxyl group of this solution with an alcoholic solution of potassium hydroxide using phenolphthalein as an indicator (hereinafter a value thus determined is referred to as an "alkali titration-based carboxyl group level"). However, the above labeling method mentioned is preferred because the alkali titration method involves the competition with hydrolysis of the polyester backbone during the titration which may lead to an unclear titration endpoint.

The "lactic acid-glycolic acid polymer" can be produced, for example, by non-catalytic dehydration polymerization condensation starting from lactic acid and glycolic acid (JP 61-28521 A) or by a ring-closure polymerization using a catalyst starting from a cyclic diester compound such as a lactide and a glycolide (Encyclopedic Handbook of Biomaterials and Bioengineering Part A: Materials, Volume 2, Marcel Dekker, Inc. 1995). While a polymer obtained by the above known ring-closure polymerization method does not always have a free carboxyl group at a terminal of the resultant polymer, the polymer can be converted into a polymer having a certain level of the carboxyl groups per unit mass by means of the hydrolysis described, for example, in EP 083925 A, and this can be used similarly.

The above "lactic acid-glycolic acid polymer having a free carboxyl group at its terminal" can be produced by a known method or method analogous thereto (for example, see JP 61-28521 A).

When an injectable preparation is produced from the microcapsules, their aqueous dispersion is prepared together with a dispersing agent (e.g., Tween 80, Eco-60, carboxymethylcellulose, sodium alginate, etc.), a preservative (e.g., methyl paraben, propyl paraben, etc.), an isotonicity (e.g., sodium chloride, mannitol, sorbitol, glucose, etc.), and the like, or their oily dispersion is prepared by dispersing together with a vegetable oil such as sesame oil and corn oil, whereby obtaining a practically usable sustained release injectable preparation.

An agent comprising the above GnRH agonist (preferably leuprorelin acetate) (preferably an agent containing a sustained release microcapsule containing leuprorelin acetate) can readily be administered as such subcutaneously, intramuscularly, intravascularly (preferably subcutaneously) in the form of an injectable preparation or an implant (preferably injectable preparation). Further, it may be administered also in the form of the above other various preparations, and can be used as a starting material for producing such preparations.

While the dosage of the above preparation may vary depending on the amount of a GnRH agonist (preferably leuprorelin acetate), dosage form, duration period of the GnRH agonist (preferably leuprorelin acetate), subject [e.g., warm-blooded mammal (e.g., human, mouse, rat, rabbit, sheep, pig, cattle, horse, etc.)], it may be a pharmaceutically effective amount of the GnRH agonist (preferably leuprorelin acetate). For example, a single dose to the warm-blooded mammal may be selected from the range of about 0.01 mg to 100 mg/kg body weight, preferably about 0.02 mg to 50 mg/kg body weight, more preferably 0.05 mg to 20 mg/kg body weight.

When the above preparation is administered as an injectable preparation, for an adult patient (weighing 60 kg) with prostate cancer, the single dose of the GnRH agonist (preferably leuprorelin acetate) is usually about 0.01 to 50 mg, preferably about 0.1 to 20 mg, more preferably about 0.1 to 15 mg, which may be administered subcutaneously or intramuscularly. When an injectable preparation containing the above sustained release microcapsule containing the GnRH agonist (preferably leuprorelin acetate) is administered, its dosage may vary depending on the release-lasting period of the drug contained in the sustained release microcapsule; for example, in case where the preparation is administered once per about one month, for an adult patient (weighing 60 kg) with prostate cancer, usually, the preparation may be administered subcutaneously or intramuscularly at a single dose of the GnRH agonist (preferably leuprorelin acetate) of about 0.01 to 20 mg, preferably about 0.1 to 10 mg, more preferably about 0.1 to 5 mg; for example, in case where the preparation is administered once per about three months, for an adult patient (weighing 60 kg) with prostate cancer, usually, the preparation was administered subcutaneously or intramuscularly at a single dose of the GnRH agonist (preferably leuprorelin acetate) of about 0.1 to 30 mg, preferably about 0.1 to 20 mg, more preferably about 1 to 15 mg.

In case of other animals, the corresponding dosage can be administered by converting the dose into that per 60 kg body weight.

By combining the above GnRH agonist with a concomitant drug, the following advantageous effects can be obtained:

(1) The dose of the GnRH agonist or the concomitant drug can be reduced in comparison with that administered each drug alone, whereby improving QOL;
(2) A synergistic effect can be obtained by combining the GnRH agonist with the concomitant drug;
(3) Add-Back therapy becomes possible by combining the GnRH agonist with the concomitant drug;
(4) MAB (maximum androgen blockade) therapy becomes possible by combining the GnRH agonist with the concomitant drug.

Hereinafter, a specific example of the concomitant drug to be combined with the GnRH agonist will be illustrated.

Receptor-type tyrosine kinase inhibitors

(A) Example

GW-2016.

(B) Target diseases

Prostate cancer, postoperative recurrence of prostate cancer or metastasis of prostate cancer.

(C) Effects

[1] MAB therapy becomes possible.
[2] Synergistic effects.
[3] Relieving side effects.
[4] Cancer therapy according to a blood level of soluble HER2 becomes possible.

Add-Back therapy means a treatment method for preventing or treating a disease whose exacerbation depends on a sex hormone (testosterone, estrogen, estradiol, and the like) by administration of a GnRH agonist to reduce a blood level of the hormone, wherein supplemental administration of the hormone or a drug equivalent thereto (hereinafter sometimes referred to as an Add-Back agent) is performed in order to relieving side effects (e.g., lowering of the amount of salts in bone) due to lowering of the hormone, i.e., the pharmacological efficacy. Preferably, oral administration is mainly employed for an Add-Back agent.

MAB therapy means a treatment method, wherein all androgenic actions in a prostate are blocked. For example, the therapy employs a surgical castration or a GnRH agonist for blocking a testes-derived androgenic action together with an anti-androgen for blocking an adrenal-derived androgenic action.

When using a GnRH agonist in combination with the concomitant drug, the administration periods of the GnRH agonist and the concomitant drug are not particularly limited, and the GNRH agonist or its pharmaceutical preparation and the concomitant drug or its pharmaceutical preparation can be administered to a subject simultaneously or at certain time intervals. The dose of the concomitant drug may be in accordance with a clinically employed dose, and selected appropriately depending on the subject, route, disease, combination, and the like.

The administration mode of the pharmaceutical composition which is a combination of the GnRH agonist and the concomitant drug (hereinafter sometimes abbreviated as the combination drug of the present invention) is not particularly limited in so far as the GnRH agonist and the concomitant drug are combined at the time of administration. Examples of such an administration mode include (1) administration of a single preparation obtained by formulating the GnRH agonist and the concomitant drug into a pharmaceutical composition simultaneously, (2) simultaneous administration via the same route of two different preparations obtained by formulating the GnRH agonist and the concomitant drug into separate pharmaceutical compositions, (3) administration via the same route of two different preparations obtained by formulating the GnRH agonist and the concomitant drug into separate pharmaceutical compositions at certain time intervals, (4) simultaneous administration via different routes of two different preparations obtained by formulating the GnRH agonist and the concomitant drug into separate pharmaceutical compositions (5) administration via different routes of two different preparations obtained by formulating the GnRH agonist and the concomitant drug into separate pharmaceutical compositions (for example, the GnRH agonist followed by the concomitant drug, and vice versa), and the like.

The above concomitant drug-containing preparation has low toxicity, and thus the concomitant drug can be safely administered orally or parenterally (e.g., topically, rectally, intravenously) by mixing it with a pharmacologically acceptable carrier in accordance with a method known per se to form a pharmaceutical preparation, for example, tablets (including sugar-coated and film-coated tablets), powders, granules, capsules (including soft capsules), solutions, injectable preparations, suppositories, sustained release preparations, and the like. An injectable preparation may be administered intravenously, intramuscularly, subcutaneously, into an organ, or directly into a lesion.

As the pharmacologically acceptable carrier which may be employed for producing the preparation, there are various organic and inorganic carrier materials conventionally employed as pharmaceutical materials and examples thereof include excipients, lubricants, binders and disintegrants for solid preparations, solvents, dissolution aids, suspending agents, isotonicities, buffers and soothing agents for liquid preparations. Further, if necessary, other additives such as conventional preservatives, antioxidants, colorants, sweeteners, adsorbents, wetting agents, etc. may also be used in suitable amounts.

Examples of the excipients include lactose, sugar, D-mannitol, starch, corn starch, crystalline cellulose, light silicic anhydride, and the like.

Examples of the lubricants include magnesium stearate, calcium stearate, talc, colloidal silica, and the like.

Examples of the binders include crystalline cellulose, sugar, D-mannitol, dextrin, hydroxypropyl cellulose, hydroxypropylmethyl cellulose, polyvinyl pyrrolidone, starch, sucrose, gelatin, methyl cellulose, sodium carboxymethylcellulose, and the like.

Examples of the disintegrants include starch, carboxymethylcellulose, calcium carboxymethylcellulose, sodium carboxymethyl starch, L-hydroxypropyl cellulose, and the like.

Examples of the solvents include injectable water, alcohol, propylene glycol, macrogol, sesame oil, corn oil, olive oil, and the like.

Examples of the dissolution aids include polyethylene glycol, propylene glycol, D-mannitol, benzyl benzoate, ethanol, trisaminomethane, cholesterol, triethanolamine, sodium carbonate, sodium citrate, and the like.

Examples of the suspending agents include surfactants such as stearyl triethanolamine, sodium lauryl sulfate, laurylaminopropionic acid, lecithin, benzalkonium chloride, benzethonium chloride, glycerin monostearate, and the like; hydrophilic polymers such as polyvinyl alcohol, polyvinyl pyrrolidone, sodium carboxymethylcellulose, methyl cellulose, hydroxymethyl cellulose, hydroxyethyl cellulose, hydroxypropyl cellulose and the like.

Examples of the isotonicities include glucose, D-sorbitol, sodium chloride, glycerin, D-mannitol, and the like.

Examples of the buffers include buffer solutions of a phosphate, acetate, carbonate, citrate, and the like.

Examples of the soothing agents include benzyl alcohol, and the like.

Examples of the preservatives include ethyl p-oxybenzoate, chlorobutanol, benzyl alcohol, phenethyl alcohol, dehydroacetic acid, sorbic acid, and the like.

Examples of the antioxidants include sulfites, ascorbic acid, $\alpha$-tocopherol, and the like.

The amount of the concomitant drug in the pharmaceutical preparation containing the concomitant drug varies depending on the dosage form, but is usually about 0.01 to 100% by weight, preferably about 0.1 to 50% by weight, more preferably about 0.5 to 20% by weight based on the entire preparation.

In case where the concomitant drug is formulated into the preparation containing the above GnRH agonist, the same amount thereof can be incorporated in the preparation.

The amount of additives such as a carrier, etc. contained in the pharmaceutical preparation containing the concomitant drug varies depending on the dosage form, but is usually about 1 to 99.99% by weight, preferably about 10 to 90% by weight based on the entire preparation.

These preparations can be produced according to a method known per se which is usually employed in a pharmaceutical process.

For example, the concomitant drug can be formulated into an injectable preparation in the form of an aqueous

9

injectable preparation together with a dispersant (e.g. Tween 80 (ATLAS POWDER COMPANY, USA), HCO60 (NIKKO CHEMICALS CO., LTD.), polyethylene glycol, carboxymethylcellulose, sodium alginate, hydroxypropylmethyl cellulose, dextrin, etc.), a stabilizer (e.g., ascorbic acid, sodium pyrosulfite, etc.), a surfactant (e.g., polysorbate 80, macrogol. etc.), a solubilizing agent (e.g., glycerin, ethanol, etc.), a buffer (e.g., phosphoric acid or its alkaline metal salt, citric acid or its alkaline metal salt, etc.), an isotonicity (e.g., sodium chloride, potassium chloride, mannitol, sorbitol, glucose, etc.), a pH adjuster (e.g., hydrochloric acid, sodium hydroxide, etc.), a preservative (e.g., ethyl p-oxybenzoate, benzoic acid, methylparabene, propylparabene, benzyl alcohol, etc.), a solubilizer (e.g., concentrated glycerin, meglumine, etc.), a solubilizing aid (e.g., propylene glycol, sugar, etc.), a soothing agent (e.g., glucose, benzyl alcohol, etc.), or in the form of an oily injectable preparation obtained by dissolving, suspending or emulsifying in a vegetable oil such as olive oil, sesame oil, cottonseed oil and corn oil, and in a solubilizing aid such as propylene glycol.

In order to obtain a preparation for oral administration, according to a method known per se, for example, the concomitant drug is admixed with an excipient (e.g., lactose, sugar, starch, etc.), a disintegrant (e.g., starch, calcium carbonate, etc.), a binder (e.g., starch, gum arabic, carboxymethylcellulose, polyvinyl pyrrolidone, hydroxypropyl cellulose, etc.) or a lubricant (e.g., talc, magnesium stearate, polyethylene glycol 6000, etc.), the mixture is compression-molded and, if necessary, the molded product is subjected to coating for taste masking, coating with an enteric coat or coating for sustained release properties by means of a method known per se to obtained the desired preparation. Examples of the coating agent to be used include hydroxypropylmethyl cellulose, ethyl cellulose, hydroxymethyl cellulose, hydroxypropyl cellulose, polyoxyehtylene glycol, Tween 80, Pluronic F68, cellulose acetate phthalate, hydroxypropylmethyl cellulose phthalate, hydroxymethyl cellulose acetate succinate, Eudragit (Rohm, Germany, methacrylic/acrylic acid copolymer), a colorant (e.g., iron oxide red, titanium dioxide), etc. The preparation for oral administration may be a rapid release preparation or a sustained release preparation.

In order to obtain, for example, a suppository, according to a method known per se, the concomitant drug can be formulated into an oily or aqueous solid, semi-solid or liquid suppository. Examples of an oily base to be used in the above composition include higher fatty acid glycerides [e.g., cocoa butter, WITEPSOL (DYNAMIT NOBEL, Germany). etc.], medium fatty acids [e.g., MIGLYOL (DYNAMIT NOBEL, Germany), etc.], or vegetable oils (e.g., sesame oil, soybean oil, cottonseed oil, etc.). Examples of an aqueous base to be used include polyethylene glycols and propylene glycol, and examples of an aqueous gel base to be used include natural gums, cellulose derivatives, vinyl polymers acrylate polymers, and the like.

As the above sustained release preparation, there is, for example, a sustained release microcapsule.

The sustained release microcapsule can be obtained by a method known per se. However, preferably, the sustained release preparation is prepared as shown in [2] hereinafter and administered.

Hereinafter, [1] an injectable preparation the concomitant drug and its production, [2] a sustained release or rapid release preparation of the concomitant drug and its production and [3] a sublingual tablet, buccal or oral rapid disintegration preparation of the concomitant drug and its production will be specifically illustrated.

[1] Injectable preparation and its production

Preferably, the injectable preparation comprises the concomitant drug dissolved in water. The preparation may further contain a benzoate and/or a salicylate.

The injectable preparation is obtained by dissolving the concomitant drug and, optionally, a benzoate and/or a salicylate in water.

Examples of the salt of the above benzoic acid and salicylic acid include those with alkali metals such as sodium and potassium, alkaline earth metals such as calcium and magnesium, an ammonium salt, a meglumine salt as well as those with organic bases such as trometamol, and the like.

The concentration of the concomitant drug in the injectable preparation is 0.5 to 50 w/v %, preferably about 3 to 20 w/v %. Preferably, the concentration of a benzoate and/or a salicylate is 0.5 to 50 w/v %, preferably 3 to 20 w/v %.

The preparation may further contain appropriate additives conventionally employed in an injectable preparation, for example, a stabilizer (ascorbic acid, sodium pyrosulfite, and the like), a surfactant (polysorbate 80, macrogol, and the like), a solubilizing agent (glycerin, ethanol, and the like), a buffer (phosphoric acid and its alkali metal salt, citric acid and its alkali metal salt, and the like), an isotonicity (sodium chloride, potassium chloride, and the like), a dispersing agent (hydroxypropylmethyl cellulose, dextrin), a pH adjuster (hydrochloric acid, sodium hydroxide, and the like), a preservative (ethyl p-oxybenzoate, benzoic acid, and the like), a solubilizer (concentrated glycerin, meglumine, and the like), a solubilizing aid (propylene glycol, sugar and the like), a soothing agent (glucose, benzyl alcohol, and the like), and the like. Each of these additives is formulated into the injectable preparation in an amount conventionally employed.

The pH of the injectable preparation is adjusted to 2 to 12, preferably 2.5 to 8.0 with a pH adjuster.

The injectable preparation can be obtained by dissolving the GnRH antagonist or the concomitant drug, optionally, a benzoate and/or salicylate, and if necessary the above additives in water. These components may be dissolved in any order as appropriate according to the same manner as a conventional production of an injectable preparation.

An injectable aqueous solution is preferably warmed, and can be used in the injectable preparation by filter sterilization or autoclave sterilization as that in a conventional production of an injectable preparation.

The injectable aqueous solution is preferably autoclaved at 100 to 121°C for 5 to 30 minutes.

An antibacterial activity may be imparted to the preparation so that it can be administered several times in divided doses.

[2] Sustained release or rapid release preparation and its production

Preferably, the sustained release preparation comprises a core containing the concomitant drug, optionally coated with a coating agent such as a water-insoluble material, a swelling polymer, etc. For example, the sustained release for oral administration which is administered once a day is preferred.

Examples of the water-insoluble material to be used as the coating agent include cellulose ethers such as ethyl cellulose, butyl cellulose, etc.; cellulose esters such as cellulose acetate, cellulose propionate, etc.; polyvinyl esters such as polyvinyl acetate, polyvinyl butyrate, etc.; acrylate polymers such as acrylic acid/methacrylic acid copolymer, methyl methacrylate copolymer, ethoxyethyl methacrylate/cinnamoethyl methacrylate/aminoalkyl methacrylate copolymer, polyacrylic acid, polymethacrylic acid, methacrylic acid alkyl amide copolymer, poly(methyl methacrylate), polymethacryl amide, aminoalkyl methacrylate copolymer, poly(methacrylic anhydride), glycidyl methacrylate copolymer, especially, a series of Eudragit (Rohm & Pharma) such as Eudragit RS-100, RL-100, RS-30D, RL-30D, RL-PO, RS-PO (ethyl acrylate/methyl methacrylate/chlorotrimethyl methacrylate/ethyl ammonium copolymer) and Eudragit NE-30D (methyl methacrylate/ethyl acrylate copolymer), etc.; hydrogenated oils such as hydrogenated castor oil (e.g., LUBRI WAX (FREUND INDUSTRIAL CO., LTD.)), etc.; waxes such as carnauba wax, fatty acid glycerin ester, paraffin, etc.; polyglycerin fatty acid esters; and the like.

As the swelling polymer, a polymer having an acidic cleavable group and exhibiting a pH-dependent swelling is preferred, and an acidic cleavable group-bearing polymer which undergoes a less swelling at an acidic pH such as in stomach but is swollen extensively at a neutral pH such as in small and large intestines is preferred.

As such a polymer having an acidic cleavable group and exhibiting a pH-dependent swelling, there are, for example, crosslinked polyacrylic acid polymers such as Carbomers 934P, 940, 941, 974P, 980, 1342 and the like, Polycarbophil and Calcium Polycarbophil (BF GOODRICH), HIGHVIS Wakos 103, 104, 105 and 304 (Wako Pure Chemical Industries, Ltd.), and the like.

The coating agent used in a sustained release preparation may further contain a hydrophilic material.

Examples of the hydrophilic material include polysaccharides which may have sulfate groups such as pullulan, dextrin and alkali metal alginates, etc.; polysaccharides having hydroxyalkyl groups or a carboxyalkyl groups such as hydroxypropyl cellulose, hydroxypropylmethyl cellulose and sodium carboxymethylcellulose, etc.; methyl cellulose; polyvinyl pyrrolidone; polyvinyl alcohol; polyethylene glycol; and the like.

The water-insoluble material content in the coating of the sustained release preparation is about 30 to about 90% (w/w), preferably about 35 to about 80% (w/w), more preferably about 40 to about 75% (w/w), and the swelling polymer content is about 3 to about 30% (w/w), preferably about 3 to about 15% (w/w). The coating may further contain the hydrophilic material and, in such a case, its content in the coating is about 50% (w/w) or less, preferably about 5 to about 40% (w/w), more preferably about 5 to about 35% (w/w). As used herein, the % (w/w) represents a % by weight based on a coating composition which is the remainder of a coating solution after removing any solvent (e.g., water, a lower alcohol such as methanol, ethanol, etc.) therefrom.

The sustained release preparation is produced by preparing a core containing a drug as exemplified hereinafter, followed by coating the resultant core with a solution of the coating agent obtained by heat-melting the water-insoluble material or the swelling polymer or by dissolving or dispersing such material in a solvent.

I. Preparation of drug-containing core

The form of a core containing drug and coated with a coating agent (hereinafter sometimes simply referred to as a core) is not specifically limited. However, preferably, it is formed in the form of particles such as granules or fine particles.

In case that a core is granules or fine particles, it has an average particle size of, preferably, about 150 to 2,000 μm, more preferably about 500 to 1,400 μm.

The core can be prepared by a conventional method. For example, the drug is admixed with suitable excipient, binder, disintegrant, lubricant, stabilizer, and the like, and then subjected to wet extrusion granulation, fluidized bed granulation, etc. to prepare the core.

The drug content in the core is about 0.5 to about 95% (w/w), preferably about 5.0 to about 80% (w/w), more preferably about 30 to about 70% (w/w).

Examples of the excipient contained in the core include saccharides such as sucrose, lactose, mannitol, glucose, etc., starch, crystalline cellulose, calcium phosphate, corn starch, and the like. Among them, crystalline cellulose and corn starch are preferred.

Examples of the binder include polyvinyl alcohol, hydroxypropyl cellulose, polyethylene glycol, polyvinyl pyrrolidone, Pluronic F68, gum arabic, gelatin, starch, and the like. Examples of the disintegrant include calcium carboxymethylcellulose (ECG505), sodium croscarmellose (Ac-Di-Sol), crosslinked polyvinyl pyrrolidone (crospovidone) a low-substituted hydroxypropyl cellulose (L-HPS), and the like. Among them, hydroxypropyl cellulose, polyvinyl pyrrolidone and a low-substituted hydroxypropyl cellulose are preferred. Examples of the lubricant and the anticoagulant include talc, magnesium stearate and its inorganic salts, and examples of the lubricant include polyethylene glycol and the like. Examples of the stabilizer include acids such as tartaric acid, citric acid, succinic acid, fumaric acid, maleic acid, etc.

In addition to the above method, the core can also be prepared by a drum granulation method wherein the drug or a mixture thereof with an excipient, a lubricant, etc. is added slowly to inert carrier particles as seeds for the core with spraying a binder dissolved in a suitable solvent such as water, a lower alcohol (e.g., methanol, ethanol, etc.), etc. as well as a pan coating method, a fluidized bed coating method and a melting granulation method. Examples of the inert carrier particles to be used include those prepared from sugar, lactose, starch, crystalline cellulose or waxes, and the average particle size thereof is preferably about 100 $\mu$m to about 1,500 $\mu$m.

In order to separate the drug contained in the core from the coating agent, the surface of the core may be covered with a protective material. As such a protective material, the above hydrophilic material and water-insoluble material can be used. The preferred protective material to be used is polyethylene glycol or polysaccharides having hydroxyalkyl groups or carboxyalkyl groups, more preferably, hydroxypropylmethyl cellulose and hydroxypropyl cellulose. The protective material may contain, as a stabilizer, an acid such as tartaric acid, citric acid, succinic acid, fumaric acid, maleic acid, etc. as well as a lubricant such as talc. In case of using the protective material, its coating build-up is about 1 to about 15% (w/w), preferably about 1 to about 10% (w/w), more preferably about 2 to about 8% (w/w) to the core.

The protective material can be coated by a conventional coating method. Specifically, it can be coated, for example, by spraying the protective material onto the core by a fluidized bed coating method, a pan coating method, etc.

II. Coating of core with coating agent

The core obtained in the above I is coated with a solution of the containing agent obtained by heat-melting the water-insoluble material or the pH-dependent swelling polymer, and the hydrophilic material or by dissolving or dispersing such material in a solvent to prepare the sustained release preparation.

As a method for coating the core with a solution of the coating agent, there is, for example, spray coating.

The composition ratio between the water-insoluble material, and the swelling polymer or the hydrophilic material in the solution of coating agent may be selected appropriately so that respective contents in the coating become those specified above.

The coating build-up of the coating agent is about 1 to about 90% (w/w) based on the core (excluding that of the protective material), preferably about 5 to about 50% (w/w), more preferably about 5 to about 35% (w/w).

The solvent for the solution of coating agent is water or an organic solvent alone, or a mixed solvent thereof. In case of using the mixed solvent, the mixing ratio between water and an organic solvent (water/organic solvent: weight ratio) may vary from 1 to 100%, and is preferably 1 to about 30%. While the organic solvent is not specifically limited in so far as it can dissolve the water-insoluble material, examples thereof to be used include a lower alcohol such as methyl alcohol, ethyl alcohol, isopropyl alcohol, n-butyl alcohol, etc.; a lower alkanone such as acetone, etc.; acetonitrile; chloroform; methylene chloride; and the like. Among them, a lower alcohol is preferred, with ethyl alcohol and isopropyl alcohol being especially preferred. Water and a mixed solvent of water and an organic solvent are preferably used as solvents for the coating agent. At this time, if necessary, an acid such as tartaric acid, citric acid, succinic acid, fumaric acid and maleic acid may be added to the solution of coating agent in order to stabilize the solution of coating agent.

The spray coating can be performed by a conventional coating method and, specifically, the core is sprayed with the solution of coating agent by a fluidized bed coating method, a pan coating method, etc. At this time, if necessary, a lubricant such as talc, titanium oxide, magnesium stearate, calcium stearate, light silicic anhydride, etc., and a plasticizer such as glycerin fatty ester, hardened castor oil, triethyl citrate, cetyl alcohol, stearyl alcohol, etc. may be added.

After coating with the coating agent, if necessary, an antistatic agent such as talc may be admixed.

The rapid release preparation may be in the form of a liquid (solution, suspension, emulsion, etc.) or a solid (particles, pill, tablet, etc.). While a preparation for oral administration and a preparation for parenteral administration such as an injectable preparation can be used, a preparation for oral administration is preferred.

Usually, the rapid release preparation may contain carriers, additives and excipients (hereinafter sometimes abbreviated as excipients) which are conventionally employed in the pharmaceutical field, in addition to the drug which is the active component. The excipient to be used is not specifically limited as far as it is a conventional excipient used in the pharmaceutical field. Examples of the excipient for the solid preparation for oral administration include lactose, starch, corn starch, crystalline cellulose (Asahi Kasei, Avicel PH101, and the like), powdered sugar, granulated sugar, mannitol, light silicic anhydride, magnesium carbonate, calcium carbonate, L-cysteine, and the like, with corn starch and mannitol being preferred. These excipients can be used alone or in combination thereof. The amount of then excipient is, for example, about 4.5 to about 99.4 w/w %, preferably about 20 to about 98.5 w/w %, more preferably about 30 to about 97 w/w % based on the total amount of the rapid release preparation.

The drug content in the rapid release preparation can be appropriately selected from the range of about 0.5 to about 95%, preferably about 1 to about 60% based on the total amount of the rapid release preparation.

In case of the rapid release solid preparation for oral administration, usually, it contains a disintegrant in addition to the above components. Examples of the disintegrant to be used include calcium carboxymethylcellulose (GOTOKUYAKUHIN, ECG505), sodium croscarmellose (for example, Asahi Kasei, Ac-Di-Sol), crospovidone (for example, BASF, KOLLIDON CL), low-substituted hydroxypropyl cellulose (SHIN-ETSU CHEMICAL CO., LTD.), carboxymethyl starch (MATSUTANI CHEMICAL INDUSTRY CO., LTD.), sodium carboxymethyl starch (KIMURASANGYO, EXPLOTAB), partial α starch (Asahi Kasei, PCS), and the like. The disintegrant to be used is a material which can disintegrate particles, for example, by contacting with water to cause water absorption or swelling; forming channels between a core-forming active component and an excipient; or the like. These disintegrants can be used alone or in combination thereof other. While the amount of a disintegrant to be incorporated may be appropriately selected according to the kind and amount of the drug to be used, the pharmaceutical design for desired release properties, etc., the content is, for example, about 0.05 to about 30 w/w %, preferably about 0.5 to about 15 w/w % based on the total amount of the rapid release preparation.

When the rapid release preparation is a solid preparation for oral administration, in addition to the above composition, the preparation may further contain conventional additives used in a solid preparation. Examples of these additives to be added include binders (for example, sucrose, gelatin, powdery gum arabic, methyl cellulose, hydroxypropyl cellulose, hydroxypropylmethyl cellulose, carboxymethylcellulose, polyvinylpyrrolidone, pullulan, dextrin, etc.), lubricants (for example, polyethylene glycol, magnesium stearate, talc, light silicic anhydride (for example, aerosil (NIPPON AEROSIL)), surfactants (for example, anionic surfactants such as sodium alkyl sulfate, etc., nonionic surfactants such as polyoxyethylene fatty ester and polyoxyethylene sorbitan fatty ester, polyoxyethylene castor oil derivatives, etc.), colorants (for example, tar colors, caramel, blood red, titanium oxide, riboflavin), and, if necessary, corrigents (for example, sweetener, flavor, etc.), adsorbents, preservatives, wetting agents, antistatic agents, and the like. As a stabilizer, an organic acid such as tartaric acid, citric acid, succinic acid, fumaric acid, etc. may be added.

Examples of the above binder to be used include preferably hydroxypropyl cellulose, polyethylene glycol, polyvinylpyrrolidone, etc.

The rapid release preparation can be prepared by mixing the above components and, if necessary, further kneading and then molding according to a conventional pharmaceutical technology. The above mixing can be performed by a conventional method, such as mixing and kneading. Specifically, when the rapid release preparation is prepared in the form of particles, the preparation can be prepared by mixing components with a vertical granulator, a universal kneader (HATAKE TEKKOSHO), a fluidized bed granulator FD-5S (POWREX CORPORATION), and the like, followed by granulation by wet extrusion granulation, fluidized bed granulation, etc. according to the same method as that for preparing the above core of the sustained release preparation.

The rapid release preparation and the sustained release preparation thus obtained as such or, after appropriately formulating into different preparations together with an excipient, etc. according to a conventional method, a combination thereof may be administered simultaneously or at certain intervals. Alternatively, both preparations as such or together with an excipient, etc. may be formulated into a single preparation for oral administration (e.g., granules, fine particles, tablets, capsules, etc.). In addition, granules or fine particles of both preparations may be prepared, followed by filling in a single capsule to obtain the preparation for oral administration.

[3] Sublingual tablet, buccal or oral rapid disintegration preparation and its production

The sublingual tablet, buccal or oral rapid disintegration preparation may be a solid preparation such as a tablet, etc., or may be an oral mucosa plaster (film).

As the sublingual tablet, buccal or oral rapid disintegration preparation is preferably a preparation containing the concomitant drug together with an excipient. Further, it may be contain auxiliary agents such as a lubricant, an isotonicity, a hydrophilic carrier, a water-dispersible polymer, a stabilizer, etc. In order to facilitate the absorption and to enhance the bioavailability, the preparation may further contain β-cyclodextrin or β-cyclodextrin derivatives (e.g., hydroxypropyl-β-cyclodextrin, etc.).

Examples of the above excipient include lactose, sugar, D-mannitol, starch, crystalline cellulose, light silicic anhydride, and the like. As the lubricant, there are, for example, magnesium stearate, calcium stearate, talc, colloidal silica, and the like, with magnesium stearate and colloidal silica being especially preferred. As the isotonicity, there are, for example, sodium chloride, glucose, fructose, mannitol, sorbitol, lactose, saccharose, glycerin, urea, and the like, with mannitol being especially preferred. Examples of the hydrophilic carrier include swelling hydrophilic carriers such as crystalline cellulose, ethyl cellulose, crosslinked polyvinyl pyrrolidone, light silicic anhydride, silicic acid, dicalcium phosphate, calcium carbonate, and the like, with crystalline cellulose (e.g., microcrystalline cellulose) being especially preferred. Examples of the water-dispersible polymer include gums (e.g., tragacanth gum, acacia gum, guar gum), alginates (e.g., sodium alginate), cellulose derivatives (e.g., methyl cellulose, carboxymethylcellulose, hydroxymethyl cellulose, hydroxypropyl cellulose, hydroxypropylmethyl cellulose), gelatin, water-soluble starch, polyacrylic acid (e.g., carbomer), polymethacrylic acid, polyvinyl alcohol, polyethylene glycol, polyvinyl pyrrolidone, polycarbophil, ascorbate palmitate salt, and the like, with hydroxypropylmethyl cellulose, polyacrylic acid, alginates, gelatin, carboxymethylcellulose, polyvinylpyrrolidone and polyethylene glycol being preferred. Hydroxypropylmethyl cellulose is especially preferred. As the stabilizer, there are, for example, cysteine, thiosorbitol, tartatic acid, citric acid, sodium carbonate, ascorbic acid, glycine, sodium sulfite, etc., with citric acid and ascorbic acid being especially preferred.

The sublingual tablet, buccal or oral rapid disintegration preparation can be produced by mixing the concomitant drug and an excipient according to a method known per se. Optionally, the above auxiliary agents such as a lubricant, an isotonicity, a hydrophilic carrier, a water-dispersible polymer, a stabilizer, a colorant, a sweetener, a preservative, etc. may also be incorporated. After mixing the above components simultaneously or at certain time intervals, the mixture is compression-molded to obtain the sublingual tablet, buccal or oral rapid disintegration preparation. If necessary, in order to obtain suitable hardness, a solvent such as water and alcohol may be used for wetting the mixture prior to or after formation of tables, followed by molding and drying to obtain the preparation.

When the preparation is molded into the oral mucosa plaster (film), the concomitant drug and the above water-dispersible polymer (preferably, hydroxypropyl cellulose, hydroxypropylmethyl cellulose), excipient, etc. are dissolved in a solvent such as water, and then the resultant solution is cast into a film. Further, additives such as plasticizers, stabilizers, antioxidants, preservatives, colorants, buffering agents, sweeteners, etc. may be added. Glycols such as polyethylene glycol, propylene glycol, etc. may be added to impart appropriate elasticity to the film, and bio-adherent polymers (e.g., polycarbophil, carbopol) may be added to enhance adhesion of the film to an oral mucosal lining. The casting can be performed by pouring the solution onto a non-adhesive surface, spreading the solution using a coating device such as a doctor blade in a uniform thickness (preferably about 10 to 1000 microns), and then drying the solution to form a film. The film thus formed is dried at room temperature or with warming, and then cut into pieces each having a desired surface area.

As the preferred oral rapid disintegration preparation, there is, for example, a rapid diffusion dosage preparation in the form of a solid network comprising the concomitant drug and a water-soluble or water-diffusible carrier which is inert to the concomitant drug. The network is obtained by sublimating a solvent from a solid composition comprising a solution of the GnRH agonist or concomitant drug in the solvent.

Preferably, the composition of the oral rapid disintegration preparation contains, in addition to the concomitant drug, a matrix-forming agent and a secondary component.

Examples of the matrix-forming agent include animal or vegetable proteins such as gelatin, dextrin, soybean, wheat and psyllium seed proteins, etc.; gummy materials such as gum arabic, guar gum, agar, xanthane gum, etc.; polysaccharides; alginates; carboxymethylcellulose; carrageenan; dextran; pectin; synthetic polymers such as polyvinylpyrrolidone, etc.; materials derived from a gelatin-gum arabic complex; and the like. Further, those also included are saccharides such as mannitol, dextrose, lactose, galactose, trehalose, etc.; cyclic saccharides such as cyclodextrin, etc.; inorganic salts such as sodium phosphate, sodium chloride, aluminum silicate, etc.; amino acids having 2 to 12 carbon atoms such as glycine, L-alanine, L-aspartic acid, L-glutamic acid, L-hydroxyproline, L-isoleucine, L-leucine, L-phenylalanine, etc.

One or more matrix-forming agents can be introduced into a solution or suspension before solidification. Such a matrix-forming agent may be present in addition to a surfactant, or may be present in the absence of a surfactant. The matrix-forming agent serves not only to form a matrix itself, but also to aid in maintaining the diffusion state of the concomitant drug in the solution or suspension.

The composition contains the secondary component such as preservatives, antioxidants, surfactants, thickening agents, colorants, pH adjusters, flavors, sweeteners or taste masking agents, and the like. Examples of the suitable colorant include red, black and yellow iron oxides, FD&C dyes available from Ellis & Everard such as FD&C Blue No.2 and FD&C Red No.40, and the like. Examples of the suitable flavor include mint, raspberry, licorice, orange, lemon, grape fruit, caramel, vanilla, cherry and grape flavor as well as a combination thereof. Examples of the suitable pH adjuster include citric acid, tartaric acid, phosphoric acid, hydrochloric acid and maleic acid. Examples of the suitable sweetener include aspartame, acesulfame K, thaumatine, etc. Examples of the suitable taste masking agent include sodium bicarbonate, ion exchange resins, cyclodextrin inclusion compounds, adsorbents and microencapsulated apomorphine.

Usually, the preparation contains the concomitant drug in an amount of about 0.1 to about 50% by weight, preferably about 0.1 to about 30% by weight, and the preferred one is a preparation (the above sublingual tablet, buccal, etc.) which allows 90% or more of the concomitant drug to be dissolved (in water) within about 1 to about 60 minutes, preferably about 1 minutes to about 15 minutes, more preferably about 2 minutes to about 5 minutes, or an oral rapid disintegration preparation which disintegrates within about 1 to about 60 seconds, preferably about 1 to about 30 seconds, more preferably about 1 to about 10 seconds after being placed in an oral cavity.

The amount of the above excipient based on the entire preparation is about 10 to about 99% by weight, preferably about 30 to about 90% by weight. The amount of β-cyclodextrin or β-Cyclodextrin derivative based on the entire preparation is about 0 to about 30% by weight. The amount of a lubricant based on the entire preparation is about 0.01 to about 10% by weight, preferably about 1 to about 5% by weight. The amount of an isotonicity based on the entire preparation is about 0.1 to about 90% by weight, preferably about 10 to about 70% by weight. The amount of a hydrophilic carrier based on the entire preparation is about 0.1 to about 50% by weight, preferably about 10 to about 30% by weight. The amount of a water-dispersible polymer based on the entire preparation is about 0.1 to about 30% by weight, preferably about 10 to about 25% by weight. The amount of a stabilizer based on the entire preparation is about 0.1 to about 10% by weight, preferably about 1 to about 5% by weight. If necessary, the above preparation may further contain additives such as colorants, sweeteners, preservatives, and the like.

While the dose of the combination drug of the present invention varies depending on the subject's age, body weight, condition, dosage form, administration mode, administration period, etc., the daily dose for example in a patient with prostate cancer (adult, body weight: about 60 kg) is as the concomitant drug about 0.01 to about 1000 mg/kg, preferably about 0.01 to about 100 mg/kg, more preferably about 0.1 to about 100 mg/kg, particularly about 0.1 to about 50 mg/kg, especially about 1.5 to about 30 mg/kg, which is administered intravenously at once or in several portions per day. It is a matter of course that the dose may vary depending on various factors as described above, and a less amount may sometimes be sufficient and an excessive amount should sometimes be required.

The concomitant drug may be used in any amount within the range causing no problematic side effects. The daily dose of the concomitant drug is not specifically limited and may vary depending on the severity of disease, subject's age, sex, body weight, susceptibility, administration period and intervals, pharmaceutical characteristics, preparation, type, kind of active component, and the daily oral dose per 1 kg body weight in a mammal is about 0.001 to 2000 mg, preferably about 0.01 to 500 mg, more preferably about 0.1 to about 100 mg as medicaments, which is usually administered once 1 to 4 portions per day.

When the combination drug or the present invention is administered, both GnRH agonist and the concomitant drug may be administered at the same time, but it is also possible that the concomitant drug is first administered and then the GnRH agonist is administered, or that the GnRH agonist is first administered and then the concomitant drug is administered. When the GnRH agonist and the concomitant drug are administered separately at a time interval, the time interval may vary depending on the active component to be administered, dosage form and administration mode. For example, when the concomitant drug is first administered, the GnRH agonist may be administered within 1 minute to 3 days, preferably 10 minutes to 1 day, more preferably 15 minutes to 1 hour after the administration of the concomitant drug. When the GnRH agonist is first administered, then the concomitant drug may be administered within 1 minute to 1 day, preferably 10 minutes to 6 hours, more preferably 15 minutes to 1 hour after the administration of the GnRH agonist.

As to amino acids, peptides, protective groups and the like of the polypeptides described herein, when they are represented by abbreviations, they are the abbreviations based on IUPAC-IUB, Commission on Biological Nomenclature, or conventionally used in the art. As to amino acids, when an optical isomer is present, the abbreviation represents its L form unless otherwise stated.

Abbreviations are as follows.

Abu: Aminobutyric acid
Aibu: 2-Aminobutyric acid
Ala: Alanine
Arg: Arginine
Gly: Glycine
His: Histidine
Ile: Isoleucine
Leu: Leucine
Met: Methionine
Nle: Norleucoine
Nval: Norvaline
Phe: Phenylalanine
Phg: Phenylglycine
Pro: Proline
(Pyr)Glu: Pyroglutamic acid
Ser: Serine
Thr: Threonine
Trp: Triptophan

Tyr: Tyrosine
Val: Valine
D2Nal: D-3-(2-Naphthyl)alanine residue
DSer(tBu): O-tert-Butyl-D-serine
DHis(ImBzl): Nim-Benzyl-D-Histidine
PAM: Phenylacetamidemethyl
Boc: t-Butyloxycarbonyl
Fmoc: 9-Fluorenylmethyloxycarbonyl
Cl-Z: 2-Chloro-benzyloxycarbonyl
Br-Z: 2-Bromo-benzyloxycarbonyl
Bzl: Benzyl
$Cl_2$-Bzl 2,6-Dichlorobenzyl
Tos: p-Toluenesulfonyl
HONb: N-Hydroxy-5-norbornene-2,3-dicarboximide
HOBt: 1-Hydroxybenzotriazole
HOOBt: 3-Hydroxy-3,4-dihydro-4-oxo-1,2,3-benzotriazine
MeBzl: 4-Methylbenzyl
Bom: Benzyloxymethyl
Bum: t-Butoxymethyl
Trt: Trityl
DNP: Dinitrophenyl
DCC: N,N'-Dicyclohexylcarbodiimide

EXAMPLES

The present invention will be further illustrated by the following Reference Examples and Example. Here, the following Example is just for reference.

Reference Example 1

Leuprorelin acetate-containing microcapsules

Leuprorelin acetate (5.8 g) was dissolved in distilled water (6.7 ml). To this was added a dichloromethane solution (138 g) containing a polylactic acid (weight average molecular weight: 15000) (51.6 g) which was separately dissolved and filtered separately was added, and the mixture was stirred and emulsified for 9 minutes with an auto-mini-mixer (about 6000 rpm) and then adjusted to 15°C. The mixture was added to a 0.1% polyvinyl alcohol (PVA) aqueous solution (13.5 l) which was previously dissolved, filtered and adjusted at the same temperature, and then the mixture was emulsified. At this time, HOMOMIC LINE FLOW (TOKUSHU KIKA KOGYO CO., LTD.) was used and the emulsification was performed at a rotation speed of the mixer of about 7000 rpm. While stirring this W/O/W emulsion gently, the solvent was removed for about 3 hours (in-water drying method).
The resultant microcapsules were sieved through a 74 μm sieve to remove coarse particles, and then separated by filtration or centrifugation. The microcapsules were washed with distilled water to remove free drug or PVA, redispersed in a small amount of water. Then, mannitol (8.7 g) was dissolved, and the mixture was sieved and freeze-dried. Drying was preformed with elevating tray temperature gradually, and continued for 69 hours at final temperature of 52°C. The mixture was sieved and grinded to obtain a microcapsule powder. By this operation, 58 g of the microcapsule powder containing 15% D-mannitol was obtained.

Reference Examples 2

Raloxifene containing tablet

| | | |
|---|---|---|
| (1) | Raloxifene | 5.0 mg |
| (2) | Table salt | 20.0 mg |
| (3) | Distilled water | To total 2 ml |

Raloxifene (5.0 mg) and table salt (20.0 mg) are dissolved in distilled water and the solution is made up to 2.0 ml with water. The solution is filtered, and filled aseptically to a 2 ml ampoule. The ampoule is sterilized and sealed tightly to obtain a injectable solution.

Reference Example 3

| (1) | Raloxifene | 50 mg |
|---|---|---|
| (2) | Lactose | 34 mg |
| (3) | Corn starch | 10.6 mg |
| (4) | Corn starch (paste) | 5 mg |
| (5) | Magnesium stearate | 0.4mg |
| (6) | Calcium carboxymethylcellulose | 20 mg |
| | Total | 120 mg |

In accordance with a conventional method, the components (1) to (6) were mixed and compressed into tablets with a tabletting machine.

Example 1

The preparation obtained in Reference Example 1 is combined with the preparation obtained in Reference Example 2 or 3.

INDUSTRIAL APPLICABILITY

By using a GnRH agonist in combination with GW-2016, excellent effects such as improvement of the preventive or therapeutic effects on prostate cancer or relief for side effects can be obtained.

**Claims**

1.  A pharmaceutical composition for use in preventing or treating prostate cancer, postoperative recurrence of prostate cancer, or metastasis of prostate cancer, which is a combination of

    (1) an acetate of the peptide represented by the formula: 5-oxo-Pro-His-Trp-Ser-Tyr-DLeu-Leu-Arg-Pro-NH-C$_2$H$_5$, and
    (2) GW-2016.

2.  The combination of

    (1) an acetate of the peptide represented by the formula: 5-oxo-Pro-His-Trp-Ser-Tyr-DLeu-Leu-Arg-Pro-NH-C$_2$H$_5$, and
    (2) GW-2016 for use in preventing or treating prostate cancer, postoperative recurrence of prostate cancer, or metastasis of prostate cancer.

3.  Use of a combination of

    (1) an acetate of the peptide represented by the formula: 5-oxo-Pro-His-Trp-Ser-Tyr-DLeu-Leu-Arg-Pro-NH-C$_2$H$_5$ and
    (2) GW-2016 for the manufacture of a medicament for the prevention or treatment of prostate cancer, postoperative recurrence of prostate cancer, or metastasis of prostate cancer.

**EP 1 424 080 B1**

**Patentansprüche**

1. Pharmazeutische Zusammensetzung zur Verwendung bei der Vorbeugung oder Behandlung von Prostatakrebs, postoperativem Wiederauftreten von Prostatakrebs oder Metastase von Prostatakrebs, welche eine Kombination von

   (1) einem Acetat des Peptids, dargestellt durch die Formel: 5-Oxo-Pro-His-Trp-Ser-Tyr-DLeu-Leu-Arg-Pro-NH-$C_2H_5$, und
   (2) GW-2016

   ist.

2. Kombination von

   (1) einem Acetat des Peptids, dargestellt durch die Formel: 5-Oxo-Pro-His-Trp-Ser-Tyr-DLeu-Leu-Arg-Pro-NH-$C_2H_5$, und
   (2) GW-2016

   zur Verwendung bei der Vorbeugung oder Behandlung von Prostatakrebs, postoperativem Wiederauftreten von Prostatakrebs oder Metastase von Prostatakrebs.

3. Verwendung einer Kombination von

   (1) einem Acetat des Peptids, dargestellt durch die Formel: 5-Oxo-Pro-His-Trp-Ser-Tyr-DLeu-Leu-Arg-Pro-NH-$C_2H_5$, und
   (2) GW-2016

   zur Herstellung eines Medikaments zur Vorbeugung oder Behandlung von Prostatakrebs, postoperativem Wiederauftreten von Prostatakrebs oder Metastase von Prostatakrebs.

**Revendications**

1. Composition pharmaceutique destinée à être utilisée dans la prévention ou le traitement du cancer de la prostate, de la récidive postopératoire du cancer de la prostate, ou de la métastase du cancer de la prostate, qui est une combinaison de

   (1) un acétate du peptide représenté par la formule : 5-oxo-Pro-His-Trp-Ser-Tyr-DLeu-Leu-Arg-Pro-NH-$C_2H_5$, et
   (2) GW-2016.

2. Combinaison de

   (1) un acétate du peptide représenté par la formule : 5-oxo-Pro-His-Trp-Ser-Tyr-DLeu-Leu-Arg-Pro-NH-$C_2H_5$, et
   (2) GW-2016 destinée à être utilisée dans la prévention ou le traitement du cancer de la prostate, de la récidive postopératoire du cancer de la prostate, ou de la métastase du cancer de la prostate.

3. Utilisation d'une combinaison de

   (1) un acétate du peptide représenté par la formule : 5-oxo-Pro-His-Trp-Ser-Tyr-DLeu-Leu-Arg-Pro-NH-$C_2H_5$, et
   (2) GW-2016 pour la fabrication d'un médicament destiné à la prévention ou au traitement du cancer de la prostate, de la récidive postopératoire du cancer de la prostate, ou de la métastase du cancer de la prostate.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4472382 A
- WO 8601105 A
- EP 1382350 A
- WO 0177107 A
- WO 9908668 A
- JP 60100516 A
- JP 62201816 A
- JP 4321622 A
- JP 6192068 A
- JP 9132524 A
- JP 9221417 A
- JP 11279054 A
- WO 99360099 A
- JP 61028521 A
- EP 083925 A

### Non-patent literature cited in the description

- **I. M. Jackson et al.** *Cancer Treatment Reviews,* 1989, vol. 16, 161-175
- **J. W. Clark et al.** *Int. J. Cancer,* 1996, vol. 65, 186-191
- **A. A. E1-Zarruk et al.** *Cancer Letters,* 1999, vol. 142, 185-193
- **G. S. Rao et al.** *Int. J. Radiation Oncology Biol. Phys.,* 2000, vol. 48, 1519-1528
- **J. Gee et al.** *European Journal of Cancer,* vol. 37, S261
- **L. Boehnke Michaud et al.** *Seminars in breast disease,* 2000, vol. 3, 100-111
- **A. J. Dowling et al.** *Cancer Treatment Reviews,* 1998, vol. 24, 283-301
- **O. Dalesio et al.** *The Lancet,* 2000, vol. 355, 1491-1498
- **D. J. George et al.** *Cancer Research,* 1999, vol. 59, 2395-2401
- Encyclopedic Handbook of Biomaterials and Bioengineering Part A: Materials. Marcel Dekker, Inc, 1995, vol. 2